# EUROPEAN PATENT SPECIFICATION

(11) **EP 1 171 410 B1**
(45) Date of publication and mention of the grant of the patent: **08.12.2004**
(21) Application number: 00907783.5
(22) Date of filing: 03.03.2000
(51) Int. Cl.: C07C 67/297, C07C 69/54, C08F 120/20, G02B 1/04

(54) **PROCESS FOR THE PREPARATION OF A DIOL**
VERFAHREN ZUR HERSTELLUNG EINES DIOLS
PROCEDE DE PREPARATION D'UN DIOL

(30) Priority: 16.04.1999 GB 9908806
(43) Date of publication of application: 16.01.2002
(73) Proprietor: CooperVision International Holding Company LP, St Michael, (BB)
(72) Inventor: HOLSTOCK, Barry, C., Surbiton, Surrey KT6 4AL (GB); GLASBEY, Trevor, Owen, Camberley, Surrey GU15 4AG (GB)
(74) Representative: Harding, Charles Thomas
(86) International application number: PCT/GB2000/000765
(87) International publication number: WO 2000/063149

(56) References cited:
- GB-A- 2 097 952
- US-A- 4 056 496
- G.M. COPPOLA: "Amberlyst-15, a superior acid catalyst for the cleavage of acetals" SYNTHESIS, no. 12, December 1984 (1984-12), pages 1021-1023, XP002140104 Geirg Thieme Verlag, Stuttgart, DE ISSN: 0039-7881
- PATENT ABSTRACTS OF JAPAN vol. 2000, no. 02, 29 February 2000 (2000-02-29) & JP 11 322675 A (MITSUBISHI RAYON CO LTD), 24 November 1999 (1999-11-24)

## Description

The present invention relates to a process for the preparation of a diol. The diol may be used subsequently used as a monomer for the preparation of a polymeric device or material such as a hemocompatible coating, medical device, water soluble polymer material, paint, water borne coating or an ocular device (such as a contact lens).

Polymers made from polymerisable monomers have wide spread applications. For example, polymers are used as additives for coating applications, such as paints and adhesives. Polymers are also used to prepare lenses, such as contact lenses.

Polymers are prepared by polymerising one or more types of polymerisable monomers, such as by emulsion polymerisation, solution polymerisation, suspension polymerisation or bulk polymerisation. The monomer(s) may be polymerised in the presence of optional ingredients such as any one of emulsifiers, stabilisers, surface active agents, initiators (such as photoinitiators), inhibitors, dispersants, oxidising agents, reducing agents, viscosity modifiers, catalysts, binders, activators, accelerators, tackifiers, plasticizers; saponification agents, chain transfer agents, surfactants, fillers, dyes, metal salts, and solvents.

There are numerous references on polymerisation of polymerisable monomers. For example, some teachings may be found in "Emulsion Polymerization: Theory and Practice" by D. C. Blackley (published by Wiley in 1975) and "Emulsion Polymerization" by F. A. Bovey *et al*. (published by Interscience Publishers in 1965). For example, a polymer can be prepared from monomers such as methyl acrylate, ethyl acrylate, butyl acrylate, 2-ethylhexyl acrylate, decyl acrylate, methyl methacrylate, ethyl methacrylate, butyl methacrylate, styrene, butadiene, ethylene, vinyl acetate, vinyl esters, C₉, C₁₀ and C₁₁ tertiary monocarboxylic acids, vinyl chloride, vinyl pyridine, vinyl pyrrolidine, vinylidene chloride, acrylonitrile, chloroprene, acrylic acid, methacrylic acid, itaconic acid, maleic acid and fumaric acid.

Examples of further teachings on polymerisation of polymerisable monomers may be found in "Vinyl and Related Polymers" by C.E. Schildknecht (New York: John Wiley & Sons 1952) and "Monomeric Acrylic Esters" by E.H. Riddle (New York: Reinhold Publishing Corp. 1954), and by A.G. Alexander (J Oil Colour Chemists' Association [1962] 45 12) and G.G. Greth and J.E. Wilson (J Appl Polymer Sci [1961] 5 135).

More recent teachings regarding polymerisation methods may be found in EP-A-0622378, EP-A-0634428, EP-A-0623632, EP-A-0635522, EP-A-0633273, EP-A-0632157, EP-A-0630908, EP-A-0630641, EP-A-0628614, EP-A-0628610, EP-A-0622449, EP-A-0626430 and EP-A-0625529.

As mentioned above, one particular application of polymers is in the preparation of lenses, especially contact lenses or intraocular lenses. Examples of teachings for the preparation of contact lenses may be found in EP-A-0359539, which discloses a method of forming a soft contact lens. Other documents that describe the preparation of contact lenses include WO-A-9502617 which discloses a contact lens made from a vinyl polymer bearing phosphonium groups, JP-A-06313009 which discloses a contact lens made from a polymer having a terminal phosphoryl-choline group, WO-A-9429756 which discloses a gas permeable ocular lens made from a block copolymer and a second polymer component, WO-A-9409042 which discloses a contact lens comprising polymer and a UV absorbing constituent, and WO-A-9211407 which discloses a tinted contact lens comprising a polymer and a dye wherein the lens is prepared by incorporating a dye into a hydrophilic polymer while the polymer is being formed. Further documents describing preparing contact lenses and the like from polymerisable monomers include EP-A-0574352, EP-A-0439394, EP-A-0378511 and EP-A-0424520.

Whilst polymers can be fairly easily prepared from polymerisable monomers there can be a problem in reliably and cheaply obtaining suitable monomers in a satisfactory pure form. In this regard, many desired polymerisable monomers are supplied with impurities. These impurities can be detrimental to the final product and so they have to be eliminated before the polymerisation reaction to form the desired polymer.

Impurities which is particularly problematic are compounds which act as cross-linkers during the polymerisation of the monomer. The presence of cross-linkers prevents and/or inhibits the formation of straight chain polymers. Moreover, the presence of cross-linkers in a monomer may prevent the solubilisation of polymers formed therefrom.

Another example of such a monomer is glyceryl methacrylate (GMA) which is a preferred monomer for preparing contact lenses. Examples of documents mentioning the use of such a monomer include US-A-5236969, JP-A-04335007, GB-A-2180243 and EP-A-0100381. There are two major problems with GMA. First, the impurities often vary from batch to batch and so make it difficult to have a standard purification protocol. Second, GMA is a very expensive monomer.

There have been attempts to prepare GMA from other monomers, such as isopropylideneglyceryl methacrylate (IPGMA). One such process for preparing GMA is disclosed in US-A-4056496 wherein the process includes reacting IPGMA with sulphuric acid and hydroquinone for a period of 16 hours.

Another process for preparing GMA, also mentioned in US-A-4056496, includes the hydrolysis of glycidyl methacrylate (GYMA) by treating GYMA with concentrated sulphuric acid for 6 days (M.F. Refojo [1965] Journal of Polymer Science 9 pp 3161-3170). This process is particularly disadvantageous because the addition of mineral acid to GMA may result in the formation of glyceryl dimethacrylate which is a cross-linker, and various other dimethacrylates.

The distillation of GMA to prepare purified GMA is also practised in the art. Distillation is however both difficult and costly. GMA is high boiling and therefore costs of distillation are high. Distillation typically gives losses of 15-20% of the product to be purified. Moreover, GMA may polymerise during distillation and valuable monomer lost. Yet further, the equipment which may be required to perform the distillation e.g. falling film evaporator, is expensive

Clearly these prior art methods are very labour intensive and include the use of hazardous chemicals, including toxic chemicals and flammable solvents, and hazardous process steps.

Further prior art methods for preparing GMA polymers are disclosed in US 4338419, FR 8207595, WO 93/0841 and Ezrielev et al, Vysokomol. Soedin, Ser. B, 20(10), 777-9, Hild, Makromol. Chem, 177, 1947-1972 (1976) and Beinert et al, Die Makromolekulare Chemie, 175,2069-2077 (1974)

US-A-5532289 discloses a process for forming a soft contact lens. According to the claims of this patent the lens is formed from a copolymer consisting essentially of 2,3-dihydroxypropyl methacrylate (i.e. glyceryl methacrylate [GMA]) and 2-hydroxyethyl methacrylate. Hydroxyethyl methacrylate is sometimes referred to as HEMA. The process of US-A-5532289 requires a pre-distillation step wherein GMA is distilled. Hence, the process of US-A-5532289 is laborious and costly.

WO 98/07055 discloses a process of preparing an ocular device (such as a contact lens) consisting essentially of GMA and HEMA, the process comprising the following steps: a) copolymerising a second monomer and a first monomer having attached to it a modifier group, thereby to form a first polymer having associated with it the modifier group; and b) modifying all or some the modifier group associated with the first polymer to form a second polymer different from the first polymer thereby to form the ocular device consisting essentially of GMA and HEMA.

The present invention seeks to overcome the problems associated with the known processes for preparing polymers.

According to a first aspect of the present invention there is provided a process for the preparation of a polymerisable monomer of formula I 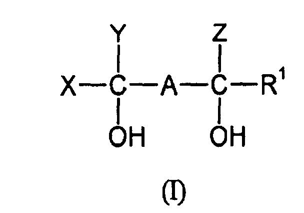 comprising the step of contacting a compound of formula II 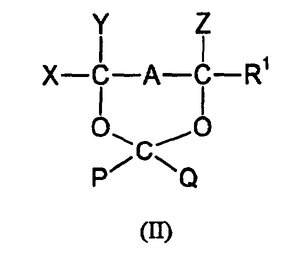 with an immobilised acid,
wherein X, Y, Z, P and Q are independently selected from a hydrocarbyl group or hydrogen, A is (CH₂)ₙ wherein n is 0 or 1 and wherein R¹ is a group of formula 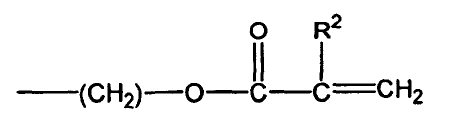 wherein R² is selected from H, methyl, ethyl, propyl and butyl or R¹ is a group of formula III 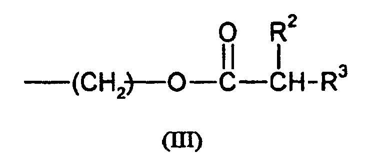 wherein R² is selected from methyl, ethyl, propyl and butyl and R³ is selected from an unsaturated C₂₋₅ alkyl.

The present invention may provide a number of advantages.

The present invention has the advantage that the presence of impurities which act as cross-linkers during the polymerisation of the monomer is reduced or avoided. Thus, the present invention provides a monomer capable of polymerisation to form a straight chain polymer without the need to purify the monomer prior to polymerisation. Moreover, the monomer is capable of polymerisation to form a polymer which may be readily solubilised.

The present invention has the advantage that because of the absence of cross-linker or the low initial cross-linker concentration in the monomer, controlled additions of further cross-linker to achieve the desired/optimum level are possible. In contrast, if monomer e.g. GMA, in accordance with the prior art is used, the level of cross-linker is often already too high to allow further additions without detriment to the mechanical properties of the final polymer, such as a lens. This advantage of the present invention is demonstrated if GMA is polymerised. The resultant polymer may simply dissolve into a suitable solvent such as water.

In a further aspect there is provided the process of the present invention for the preparation of a composition comprising a polymerisable monomer of formula I.

In a preferred aspect of the process of the present invention the acid is a strong acid. By the term "strong acid" it is meant an acid having a pKa of less than 3.

In a preferred aspect of the process of the present invention the acid is immobilised on an ion exchange resin. More preferably the acid is a strong acid immobilised on an ion exchange resin. Yet more preferably the ion exchange resin is Amberlyst 15, obtainable from Röhm and Haas of USA.

The term "hydrocarbyl group" as used herein means a group comprising at least C and H and may optionally comprise one or more other suitable substituents. Examples of such substituents may include halo-, alkoxy-, nitro-, hydroxy, carboxyl, epoxy, acrylic, hydrocarbon, N-acyl, or cyclic group etc. In addition to the possibility of the substituents being a cyclic group, a combination of substituents may form a cyclic group. If the hydrocarbyl group comprises more than one C then those carbons need not necessarily be linked to each other. For example, at least two of the carbons may be linked via a suitable element or group. Thus, the hydrocarbyl group may contain hetero atoms. Suitable hetero atoms will be apparent to those skilled in the art and include, for instance, sulphur, nitrogen and oxygen.

Preferably, the hydrocarbyl group is a linear or a branched group. The branched group may contain one or more branches.

In one aspect, the linear or a branched hydrocarbyl group may contain from 1 to 20 carbon atoms, from 1 to 15 carbon atoms, from 1 to 10 carbon atoms, from 1 to 5 carbon atoms, or from 1 to 3 carbon atoms.

The linear or a branched hydrocarbyl group may be saturated or unsaturated. In one aspect, the linear or a branched hydrocarbyl group may saturated.

Preferably, the hydrocarbyl group comprises an alkyl backbone. The backbone may be interrupted by one or more non-alkyl groups. The non-alkyl groups may be selected from ester, ether and combinations thereof.

In one aspect, the alkyl backbone may contain from 1 to 20 carbon atoms, from 1 to 15 carbon atoms, from 1 to 10 carbon atoms, from 1 to 5 carbon atoms, or from 1 to 3 carbon atoms.

The alkyl backbone may contain one or more alkyl groups branched from the alkyl backbone. In one aspect, each alkyl branch may contain from 1 to 20 carbon atoms, from 1 to 15 carbon atoms, from I to 10 carbon atoms, from 1 to 5 carbon atoms, or from 1 to 3 carbon atoms.

The hydrocarbyl group may be a cyclic group. In this regard, the hydrocarbyl group can be a single ring group or a polycyclic group. Here, the term "polycyclic" includes fused and non-fused ring structures including combinations thereof.

At least one of the cyclic groups of the polycyclic group may be a heterocyclic group (a heterocycle) or a non-heterocyclic group.

The cyclic group or at least one of the cyclic groups of the polycyclic group may be a saturated ring structure or an unsaturated ring structure (such as an aryl group).

The hydrocarbyl group may contain any one or more of C, H, O, Si, N, P, halogen (including Cl, Br and I), S and P. In the preferred aspect wherein the hydrocarbyl group comprises an alkyl backbone, the alkyl backbone may be interrupted by one or more of any one or more of C, H, O, Si, N, P, halogen (including Cl, Br and I), S and P.

Preferably, the hydrocarbyl group is a hydrocarbon group.

Here the term "hydrocarbon group" means any one of an alkyl group, an alkenyl group, an alkynyl group, an acyl group, which groups may be linear, branched or cyclic, or an aryl group. The term hydrocarbon also includes those groups but wherein they have been optionally substituted. If the hydrocarbon is a branched structure having substituent(s) thereon, then the substitution may be on either the hydrocarbon backbone or on the branch; alternatively the substitutions may be on the hydrocarbon backbone and on the branch.

The hydrocarbon group may have from 1 to 20 carbon atoms, from 1 to 15 carbon atoms, from 1 to 10 carbon atoms, from 1 to 5 carbon atoms, or from 1 to 3 carbon atoms.

As previously mentioned, R¹ is a group of formula 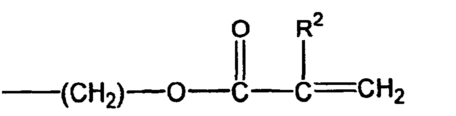 wherein R² is selected from H, methyl, ethyl, propyl and butyl; or R¹ is a group of formula III 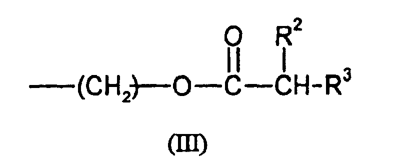 wherein R² is selected from methyl, ethyl, propyl and butyl and R³ is selected from an unsaturated C₂₋₅ alkyl. In a highly pleferred embodiment, R¹ is a group of formula 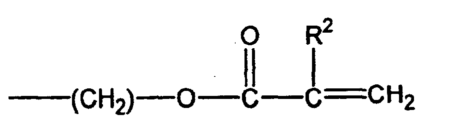 wherein R² is CH₃.

Preferably, X, Y, P and Q of formulae given in the present specification are independently selected from a hydrocarbyl group and hydrogen. Preferably, the hydrocarbyl group is a hydrocarbon group. More preferably the hydrocarbyl group is a hydrocarbon group having from 1 to 20 carbons. Yet more preferably, the hydrocarbyl group is selected from methyl, ethyl, propyl and butyl.

In a highly preferred embodiment X is H, Y is H, Z is H, P is CH₃, Q is CH₃, n=0, and R' is a group of formula 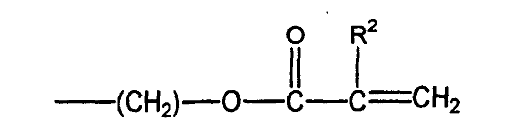 in which R² is CH₃.

Thus in a highly preferred aspect the present invention provides a process for the preparation of a polymerisable monomer of formula I 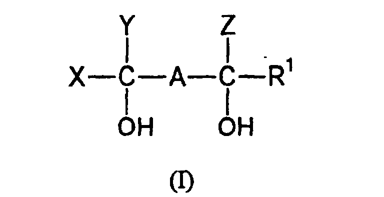 comprising the step of contacting a compound of formula II 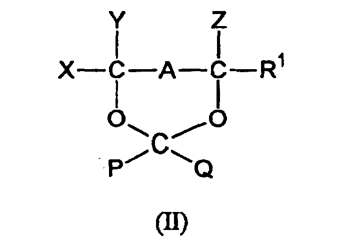 with an immobilised acid,
wherein X is H
YisH
ZisH
A is (CH₂)₀
P is CH₃
Q is CH₃
and wherein R¹ is a group of formula 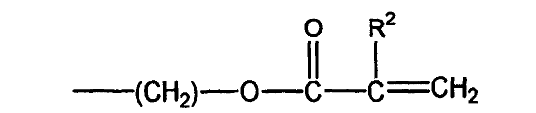 in which R² is CH₃.

In other words, in a highly preferred aspect, the present invention provides a process for the preparation of glyceryl methacrylate (GMA), comprising the step of contacting (2,2 1,3-dioxolan-4-yl) methyl methacrylate (GMAK) with an immobilised acid.

In a preferred embodiment of the present invention the process further comprises providing means for containing the immobilised acid, contacting the immobilised acid with the compound of formula II and passing a gas through the immobilised acid.

Preferably the gas contains oxygen. More preferably, the gas is air.

In a preferred embodiment of the present invention the immobilised acid is contacted with the compound of formula II in the absence of an organic solvent.

Preferably the means for containing the immobilised acid comprises a fluidised bed reactor.

In a further preferred aspect of the present invention the process comprises extracting the gas from the means for containing the immobilised acid after the gas has passed through the immobilised acid. The extracted gas may contain a reaction product of the contact of the compound of formula II with the immobilised acid. The reaction product may be of the formula P-C(O)-Q. By removing the reaction product, the equilibrium II 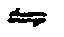 I + P-C(O)-Q will be driven towards I. Thus the rate of conversion and/or ultimate conversion of I will be increased. An essentially quantitative conversion may be obtained.

In a preferred aspect the process of the present invention comprises the step of polymerising the polymerisable monomer of formula I. Any typical, suitable polymerisation method may be used. The preferred method is free radical polymerisation, thermal or UV initiated.

In a preferred aspect the process of the present invention further comprises the step of polymerising the polymerisable monomer of formula I to provide a medical device, in particular an ocular device such as a contact lens.

The present invention is very advantageous for preparing ocular devices, such as contact lenses (both hard and soft contact lenses), intraocular lenses, interocular lenses and intercomeal implants, as well as prostheses and hydrogel articles. In this regard, the present invention not only enables the ocular devices to be made more easily but also it allows a greater control over any one of the shrinkage, the dimensional consistency, the swell, the water sensitivity, the hydrophobicity or the hydrophilicity, or combinations thereof, of the resultant polymer.

The medical device and/or polymerisable monomer and/or composition may also comprise conventional additional components such as any one or more of emulsifiers, stabilisers, surface active agents, initiators (such as photoinitiators), inhibitors, dispersants, oxidising agents, reducing agents, viscosity modifiers, catalysts, binders, activators, accelerators, tackifiers, plasticizers, saponification agents, chain transfer agents, cross-linking agents, surfactants, fillers, dyes, metal salts, and solvents.

By way of example, the surfactants and dispersants can be salts of fatty rosin and naphthenic acids, condensation products of naphthalene sulphonic acid and formaldehyde of low molecular weight, carboxylic polymers and copolymers of the appropriate hydrophile-lipophile balance, higher alkyl sulfates, such as sodium lauryl sulfate, alkyl aryl sulfonates, such as dodecylbenzene sulfonate, sodium or potassium isopropylbenzene sulfonates or isopropylnaphthalene sulfonates; sulfosuccinates, such as sodium dioctylsulfosuccinate alkali metal higher alkyl sulfosuccinates, e.g. sodium octyl sulfosuccinate, sodium N-methyl-N-galmitoyl-taurate, sodium oleyl isethionate, alkali metal salts of alkylarylpolyethoxyethanol sulfates or sulfonates, e.g. sodium t-octylphenoxy-polyethoxyethyl sulfate having 1 to 5 oxyethylene units. Typical polymerisation inhibitors that can be used include hydroquinone, monomethyl ether, benzoquinone, phenothiazine and methylene blue.

In a preferred embodiment the dye is selected from the group consisting of 2-hydroxybenzophenone, oxidiazoles, salicylic acid, resorcinol monobenzoate, benzotriazole, preferably 2H-benzotriazole, benzothiazoloazine, preferably 2N-benzothi-azoloazine, α-cyano-β-phenylcinnamic acid, polyalkypiperidine and derivatives thereof.

Preferably, the dye is selected from benzotriazole, in particular 2H-benzotriazole and derivatives thereof.

The composition and/or medical device of the present invention may comprise one or more additional comonomers. Examples of the one or more additional comonomers that can be used in the present invention include one of: (alkyl and cycloalkyl) acrylates; (alkyl and cycloalkyl) methacrylates; free-radical polymerisable olefinic acids, including alkoxy-, alkylphenoxy-, alkylphenoxy-(polyethyleneoxide)-, vinyl ester-, amine substituted (including quaternary ammonium salts thereof), nitrile-, halo-, hydroxy-, and acid substituted (for example phospho- or sulpho-) derivatives thereof; and other suitable ethylenically unsaturated polymerisable moieties; including combinations thereof. Preferably the alkyl and cycloalkyl groups contain up to 20 carbon atoms, e.g. (C₁-C₂₀ alkyl and C₁-C₂₀ cycloalkyl) acrylates, and (C₁-C₂₀ alkyl and C₁-C₂₀ cycloalkyl) methacrylates. In more detail, typical comonomers include any one of methyl acrylate, ethyl acrylate, n-propyl acrylate, isopropyl acrylate, n-butyl acrylate, isobutyl acrylate, t-butyl acrylate, isobomyl acrylate, pentyl acrylate, hexyl acrylate, octyl acrylate, iso-octyl acrylate, nonyl acrylate, lauryl acrylate, stearyl acrylate, eicosyl acrylate, 2-ethylhexyl acrylate, cyclohexyl acrylate, cycloheptyl acrylate, methyl methacrylate, ethyl methacrylate, hydroxymethylacrylate, hydroxymethylmethacrylate, propyl methacrylate, n-butyl methacrylate, t-butyl methacrylate, isobutyl methacrylate, pentyl methacrylate, hexyl methacrylate, cyclohexyl methacrylate, 2-ethylhexyl methacrylate, isobornyl methacrylate, heptyl methacrylate, cycloheptyl methacrylate, octyl methacrylate, iso-octyl methacrylate, nonyl methacrylate, decyl methacrylate, lauryl methacrylate, eicosyl methacrylate, dodecyl acrylate, pentadecyl acrylate, cetyl acrylate, stearyl acrylate, eicosyl acrylate, isodecyl acrylate, vinyl stearate, nonylphenoxy-(ethyleneoxide)₁₋₂₀ acrylate, octadecene, hexadecene, tetradecene, dodecene, dodecyl methacrylate, pentadecyl methacrylate, cetyl methacrylate, stearyl methacrylate, eicosyl methacrylate, isodecyl methacrylate, nonylphenoxy-(ethyleneoxide)₁₋₂₀ methacrylate, acrylic acid, methacrylic acid, fumaric acid, crotonic acid, itaconic acid, fumaric anhydride, crotonic anhydride, itaconic anhydride, maleic acid, maleic anhydride, styrene, alpha-methyl styrene, vinyl toluene, acrylonitrile, methacrylonitrile, ethylene, vinyl acetate, vinyl chloride, vinylidene chloride, acrylamide, methacrylamide, methacrylamide 2-cyanoethyl acrylate, 2-cyanoethyl methacrylate, dimethylaminoethyl methacrylate, dimethylaminopropyl methacrylate t-butylaminoethyl methacrylate, glycidyl acrylate, glycidyl methacrylate, glyceryl acrylate, glyceryl methacrylate, benzyl acrylate, benzyl methacrylate, phenyl acrylate, phenyl methacrylate, vinyl pyridine, vinyl pyrrolidine, siloxanes, silanes and mixtures thereof. Other polymerisable monomers are disclosed in US-A-2879178, US-A-3037006, US-A-3502627, US-A-3037969 and US-A-3497485.

Preferred comonomers include any one of glyceryl methacrylate (GMA), (2,2 dimethyl-1,3-dioxolan-4-yl) methyl methacrylate (GMAK), hydroxy ethyl methacrylate (HEMA), methacrylic acid, acrylic acid, GYMA, N-vinyl pyrrolidone, alkyl methacrylates (such as C₁₋₂₀ alkyl methacrylates, more preferably C₁₋₁₅ alkyl methacrylates, more preferably C₁₋₁₀ alkyl, methacrylates, more preferably C₁₋₅ alkyl methacrylates, such as methyl methacrylate), alkyl acrylates (such as C₁₋₂₀ alkyl acrylates, more preferably C₁₋₁₅ alkyl acrylates, more preferably C₁₋₁₀ alkyl acrylates, more preferably C₁₋₅ alkyl acrylates, such as methyl acrylate), aryl methacrylates, aryl acrylates, diacetone acrylamide, acrylamide, methacrylamide, N-alkyl acrylamides (such as C₁₋₂₀ N-alkyl acrylamides, more preferably C₁₋₁₅ N-alkyl acrylamides, more preferably C₁₋₁₀ N-alkyl acrylamides, more preferably C₁₋₅ N-alkyl acrylamides, such as methyl acrylamide), N-alkyl methacrylamides (such as C₁₋₂₀ N-alkyl methacrylamides, more preferably C₁₋₁₅ N-alkyl methacrylamides, more preferably C₁₋₁₀ N-alkyl methacrylamides, more preferably C₁₋₅ N-alkyl methacrylamides, such as methyl methacrylamide), vinyl acetate, vinyl esters, styrene, other substituted olefins, N-dialkyl acrylamides (such as C₁₋₂₀ N-dialkyl acrylamides, more preferably C₁₋₁₅ N-dialkyl acrylamides, more preferably C₁₋₁₀ N-dialkyl acrylamides, more preferably C₁₋₅ N-dialkyl acrylamides, such as N N dimethyl acrylamide), N-dialkyl methacrylamides (such as C₁₋₂₀ N-dialkyl methacrylamides, more preferably C₁₋₁₅ N-dialkyl methacrylamides, more preferably C₁₋₁₀ N-dialkyl methacrylamides, more preferably C₁₋₅ N-dialkyl methacrylamides, such as N N dimethyl methacrylamide), 3-methacryloxypropyl tris (trimethysilyl siloxy) silane (TRIS monomer), fluoro substituted alkyl and aryl acrylates and methacrylates (preferably wherein the alkyl is C₁₋₂₀ alkyl, more preferably C₁₋₁₅ alkyl, more preferably C₁₋₁₀ alkyl, more preferably C₁₋₅ alkyl), and combinations thereof.

More preferred comonomers include any one of glyceryl methacrylate (GMA), (2,2 dimethyl-1,3-dioxolan-4-yl) methyl methacrylate (GMAK), 2-hydroxy ethyl methacrylate (2-HEMA), methacrylic acid and acrylic acid, or cpmbinations thereof.

The lists of comonomers also include substituted derivatives of those monomers, such as halogenated monomers, especially fluorinated monomer derivatives, and acetal and ketal derivatives.

The polymerisable monomer of the present invention and the one or more additional comonomers may be selected so that the composition and/or ocular device of the present invention consists essentially of GMA and HEMA.

In a preferred aspect the present invention also provides an ocular device (such as a contact lens) obtained by the process of the present invention wherein the ocular device comprises HEMA in amounts of from 80-20%; GMA in amounts of from 20-80% by weight; and optionally a cross-linking polymerised monomer in an amount of 5% or less; and wherein the ocular device contains less than 0.01% methacrylic acid.

In a preferred aspect of the present invention the compound of formula II is a derivative of GMA such as (2,2-dimethyt-1,3-dioxolan-4-yl) methyl methacrylate (GMAK). GMAK can be prepared following the teachings of Mori *et al* ([1994] Macromolecules 27 pp 35-39) and Oguchi *et al* Polym Eng. Sci. ([1990] 30 449). This preferred process of the present invention is also of particular interest as GMAK can be readily synthesised from cheap, commercially available materials and it can be prepared in a pure state, i.e. free from the substances normally present in commercial GMA, especially cross-linking substances.

Thus, in one aspect of the present invention the compound of formula II is or is part of a polymer. However, as explained above, the compound of formula II is preferably a monomer.

A polymer prepared in accordance with the present invention may be fabricated as buttons or cast moulded or spun cast lenses.

When a compound of formula II is contacted with an immobilised acid to provide a polymerisable monomer of formula I, an acid may be formed by the reaction. For example, when (2,2 dimethyl-1,3-dioxolan-4-yl) methyl methacrylate (GMAK) is contacted with immobilised acid to provide GMA, methacrylic acid is formed in small amounts. In a preferred aspect of the present invention, the acid is not subsequently neutralised. In an alternative preferred aspect, wherein the present invention provides a composition comprising a polymerisable monomer of formula I, the process further comprises the step of polymerising the polymerisable monomer of formula I, with the proviso that the polymerisable monomer composition is not neutralised prior to polymerisation. In preferred aspects of the present invention, the acid is methacrylic acid or acrylic acid.

The omission of the neutralisation of any acid avoids and/or reduces the formation of cross-linker. For example, in the aspect in the present invention described above wherein GMAK is contacted with an immobilised acid to form GMA, if methacrylic acid present in the composition comprising GMA is neutralised, glycerol dimethacrylate is formed (DGMA). Glycerol dimethacrylate is a cross-linker.

Thus in a further aspect, the present invention provides a process for the preparation of a polymer, comprising the steps of
(i) contacting a compound of formula II 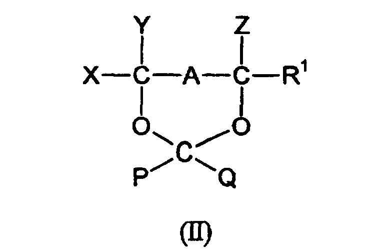 with an immobilised acid,
   wherein X, Y, Z, P and Q are independently selected from a hydrocarbyl group or hydrogen, A is (CH₂)ₙ wherein n is 0 or 1 and wherein R¹ is a group of formula 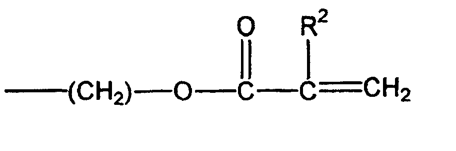 wherein R² is selected from H, methyl, ethyl, propyl and butyl; or R¹ is a group of formula III 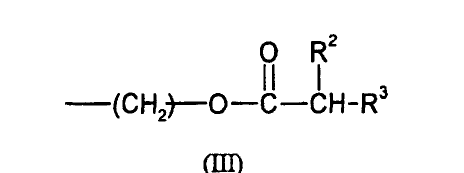 wherein R² is selected from methyl, ethyl, propyl and butyl and R³ is selected from an unsaturated C₂₋₅ alkyl;
   such that a polymerisable monomer of formula I is provided 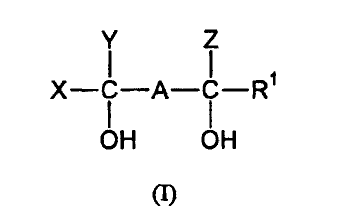 and
(ii) polymerising the polymerisable monomer of formula I, with the proviso that any acid present in the polymerisable composition is not neutralised prior to polymerisation.

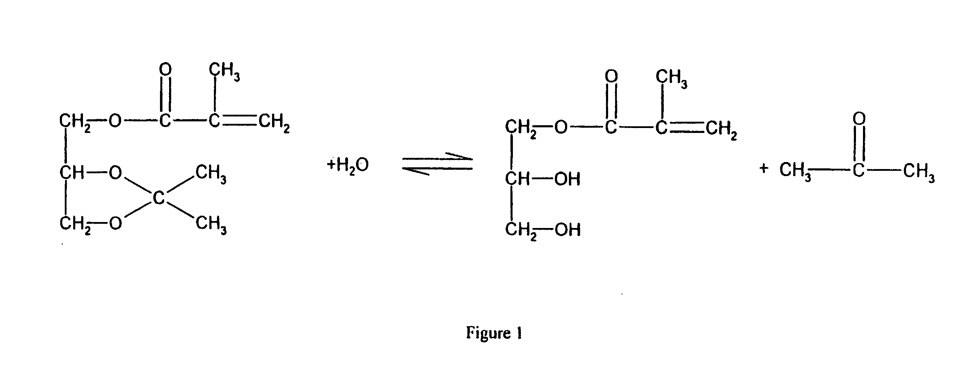

The invention will now be described, by way of example only, with reference to the accompanying drawings in which:-
Figure 1 shows a reaction scheme of a preferred process in accordance with the present invention.

### EXAMPLES

### 1. Conversion of GMAK To Glycerine Methacrylate (GMA)

The method involves the acid catalysed hydrolysis of the ketal (GMAK) with a strong acid cation exchange resin.

### Reagents Employed

| | |
|---|---|
| GMAK (water washed) | 2000 ml |
| Deionised water | 640 ml |
| Amberlyst 15 (wet) cation exchange resin | 200 g |

GMAK was obtained was prepared by transesterification of methyl methacrylate and Solketal (2,2-dimethyl-4-hydroxymethyl-1,3-dioxolane).

Before use the Amberlyst 15 (wet) was repeatably washed with deionised water to remove acidic impurities.

Before use the GMAK is washed with its own volume of deionised water to remove any Solketal. If the GMAK contains a high concentration of Solketal separation will be difficult. In this case, addition of further deionised water will effect easier separation.

The reagents are placed in a 3 litre amber glass bottle and a slow stream (6 litre/min) of filtered air passed through the mixture for 48 hours.

The cation exchange resin is then filtered off using a Buchner funnel and flask. At this stage the GMA typically contains 20 to 22% water and 0.1% methacrylic acid.

The filtrate is transferred to a 2 litre amber glass bottle and a rapid stream of dry filtered air passed through to reduce the water content to < 2%. 50ppm of MeHQ is dissolved in the resulting monomer which has a methacrylic acid content of <0.1%.

### 2. Cast Moulding

10g of GMA (prepared in Example 1) was blended with 10g of HEMA. Azoisobutyronitrile (AIBN) (0.2%) was dissolved in the mixture which was then filtered. The mixture was charged into polypropylene moulds which were assembled and heated to 120°C for 30 minutes. The cured lenses were ejected and equilibrated in saline solution. The resulting hydrogel lenses had a water content of 60%.

### 3. Spin Casting

10g of GMA (prepared in Example 1) was blended with 10g of HEMA. Benzoir methyl ether (0.2%) was dissolved in the mixture which was then filtered. The mixture was charged into PVC moulds designed for spin casting. After passage through a bank of UV lamps the cured lenses were removed from the moulds by exposure to warm water and then equilibrated in saline.

### 4. Buttons

10g of GMA (prepared in Example 1) was blended with 10g of HEMA. Isopropyl per dicarbonate (0.1%) was dissolved in the mixture which was then filtered. The mixture was charged into polypropylene button moulds which were sealed and immersed in a water bath. After 16 hours at 32° C the clear colourless buttons were ejected and heated to 120°C for 1 hour and allowed to cool to 50°C at a rate of 17°C/hr. Lenses were cut from the buttons and equilibrated in saline.

## Claims

1. A process for the preparation of a polymerisable monomer of formula I 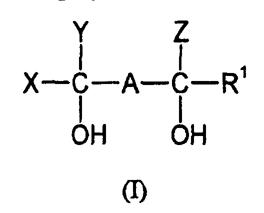 comprising the step of contacting a compound of formula II 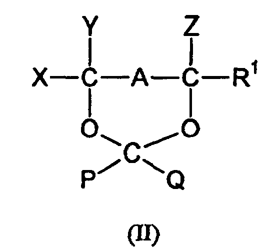 with an immobilised acid,
wherein X, Y, Z, P and Q are independently selected from a hydrocarbyl group or hydrogen, A is (CH₂)ₙ wherein n is 0 or 1 and wherein R¹ is a group of formula 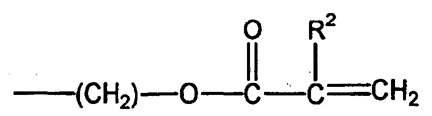 wherein R² is selected from H, methyl, ethyl, propyl and butyl; or R¹ is a group of formula III 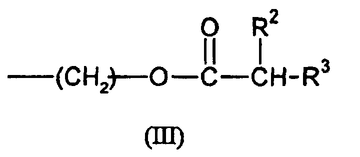 wherein R² is selected from methyl, ethyl, propyl and butyl and R³ is selected from an unsaturated C₂₋₅ alkyl.

2. A process according to claim 1 wherein the acid is a strong acid.

3. A process according to claim 1 or 2 wherein the acid is immobilised on an ion exchange resin.

4. A process according to any one of the preceding claims wherein X and Y are independently selected from hydrocarbon groups having from 1 to 20 carbon atoms and hydrogen.

5. A process according to any one of the preceding claims wherein R¹ is a group of formula 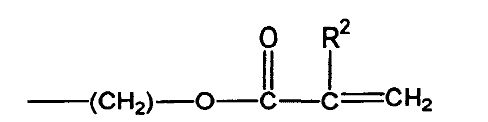 wherein R² is CH₃.

6. A process according to any one of the preceding claims wherein X is H, Y is H, Z is H, P is CH₃, Q is CH₃, n=0, R¹ is a group of formula 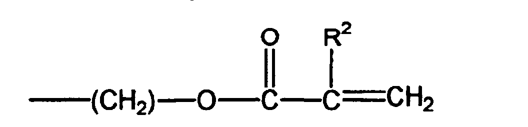 in which R² is CH₃.

7. A process according to any one of the preceding claims comprising providing means for containing the immobilised acid, contacting the immobilised acid with the compound of formula II and passing a gas through the immobilised acid.

8. A process according to claim 7 wherein the gas is air.

9. A process according to claim 7 or 8 wherein the immobilised acid is contacted with the compound of formula II in the absence of an organic solvent.

10. A process according to any one of claims 7 to 9 wherein the means for containing the immobilised acid comprises a fluidised bed reactor.

11. A process according to any one of claims 7 to 10 wherein the process comprises extracting the gas from the means for containing the immobilised acid after the gas has passed through the immobilised acid.

12. A process according to any one of the preceding claims wherein the process further comprises the step of polymerising the polymerisable monomer of formula I.

13. A process according to claim 12 wherein acid formed during the process is not neutralised prior to polymerisation.

14. A process according to claim 13 wherein the acid formed is selected from methacrylic acid and acrylic acid.

15. A process according to claim 12, 13 or 14 further comprising forming an ocular device from the polymer.

16. A process for the preparation of a polymer, comprising the steps of
(i) contacting a compound of formula II 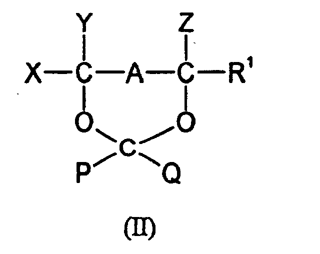 with an immobilised acid,
wherein X, Y, Z, P and Q are independently selected from a hydrocarbyl group or hydrogen, A is (CH₂)ₙ wherein n is 0 or 1 and wherein R¹ is a group of formula 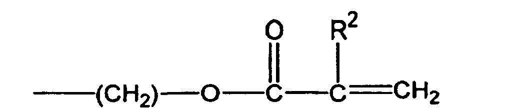 wherein R² is selected from H, methyl, ethyl, propyl and butyl or R¹ is a group of formula III 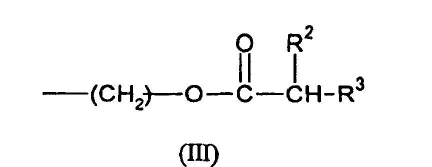 wherein R² is selected from methyl, ethyl, propyl and butyl and R³ is selected from an unsaturated C₂₋₅ alkyl;
such that a polymerisable monomer of formula I is provided 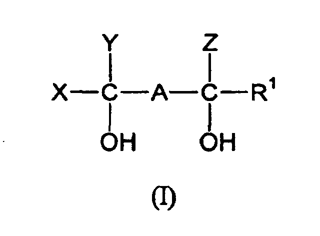 and
(ii) polymerising the polymerisable monomer of formula I, with the proviso that any acid present in the polymerisable composition is not neutralised prior to polymerisation.

## Patentansprüche

1. Verfahren zur Herstellung eines polymerisierbaren Monomers der Formel I 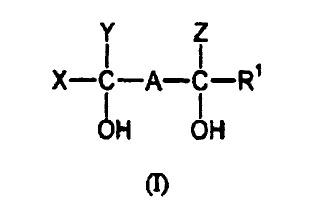 mit den Stufen, in denen man eine Verbindung der Formel II 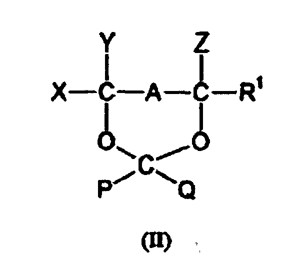 mit einer immobilisierten Säure in Kontakt bringt,
wobei X, Y, Z, P und Q unabhängig voneinander unter einer hydrocarbylgruppe oder Wasserstoff ausgewählt sind, A (CH₂)ₙ ist, wobei n 0 oder 1 ist, und wobei R¹ eine Gruppe der Formel 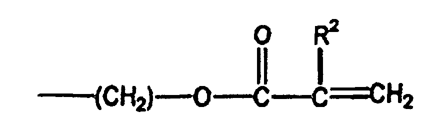 ist, wobei R² unter H, Methyl, Ethyl, Propyl und Butyl ausgewählt ist, oder R¹ eine Gruppe der Formel III 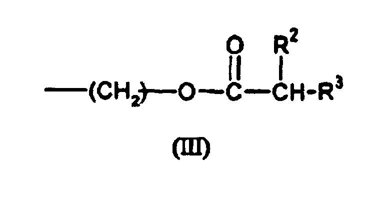 ist, wobei R² unter Methyl, Ethyl, Propyl und Butyl ausgewählt ist und R³ unter einem ungesättigten C₂₋₅-Alkyl ausgewählt ist.

2. Verfahren nach Anspruch 1, wobei die Säure eine starke Säure ist.

3. Verfahren nach Anspruch 1 oder 2, wobei die Säure an einem Ionenaustauscherharz immobilisiert ist.

4. Verfahren nach einem der vorangegangenen Ansprüche, wobei X und Y unabhängig voneinander unter Kohlenwasserstoffgruppen mit 1 bis 20 Kohlenstoffatomen und Wasserstoff ausgewählt sind.

5. Verfahren nach einem der vorangegangenen Ansprüche, wobei R' eine Gruppe der Formel 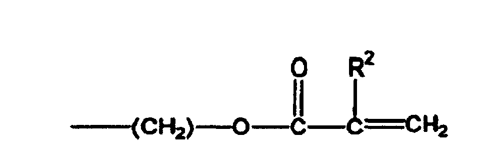 ist, wobei R² CH₃ ist.

6. Verfahren nach einem der vorangegangenen Ansprüche, wobei X H ist, Y H ist, Z H ist, P CH₃ ist, Q CH₃ ist, n = 0 und R¹ eine Gruppe der Formel 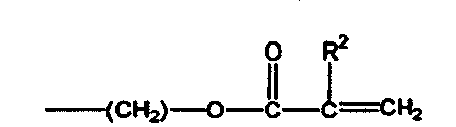 ist, in weicher R² CH₃ ist.

7. Verfahren nach einem der vorangegangenen Ansprüche, welches umfaßt, daß man eine Einrichtung bereitstellt, welche die immobilisierte Säure enthält, die immobilisierte Säure mit der Verbindung der Formel II in Kontakt bringt und ein Gas durch die immobilisierte Säure hindurchleitet.

8. Verfahren nach Anspruch 7, wobei das Gas Luft ist.

9. Verfahren nach Anspruch 7 oder 8, wobei die immobilisierte Säure mit der Verbindung der Formel II in Abwesenheit eines organischen Lösungsmittels in Kontakt gebracht wird.

10. Verfahren nach einem der Ansprüche 7 bis 9, wobei die Einrichtung, welche die immobilisierte Säure enthält, einen Wirbelschichtreaktor umfaßt.

11. Verfahren nach einem der Ansprüche 7 bis 10, wobei das Verfahren umfaßt, daß man das Gas von der Einrichtung, welche die immobilisierte Säure enthält, abzieht, nachdem das Gas durch die immobilisierte Säure hindurchgeleitet wurde.

12. Verfahren nach einem der vorangegangenen Ansprüche, wobei das Verfahren weiterhin die Stufe umfaßt, bei der man das polymerisierbare Monomer der Formel I polymerisiert.

13. Verfahren nach Anspruch 12, wobei Säure, die bei dem Verfahren gebildet wird, vor der Polymerisation nicht neutralisiert wird.

14. Verfahren nach Anspruch 13, wobei die gebildete Säure unter Methacrylsäure und Acrylsäure ausgewählt ist.

15. Verfahren nach Anspruch 12, 13 oder 14, welches weiterhin umfaßt, daß man aus dem Polymer eine Okularvorrichtung herstellt.

16. Verfahren zur Herstellung eines Polymers mit den Stufen, in denen man
(i) eine Verbindung der Formel II 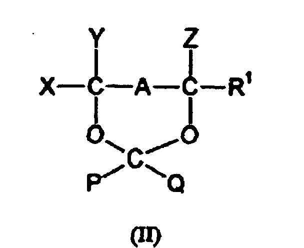 mit einer immobilisierten Säure in Kontakt bringt,
wobei X, Y, Z, P und Q unabhängig voneinander unter einer Hydrocarbylgruppe oder Wasserstoff ausgewählt sind, A (CH₂)ₙ ist, wobei n 0 oder 1 ist, und wobei R¹ eine Gruppe der Formel 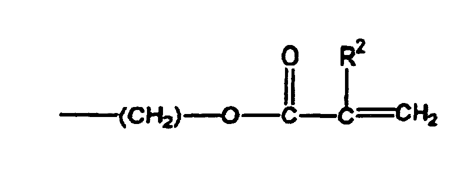 ist, wobei R² unter H, Methyl, Ethyl, Propyl und Butyl ausgewählt ist, oder R¹ eine Gruppe der Formel III 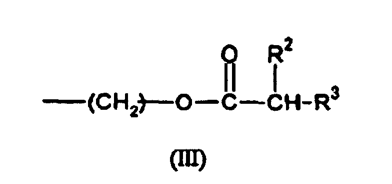 ist, wobei R² unter Methyl, Ethyl, Propyl und Butyl ausgewählt ist und R³ unter einem ungesättigten C₂₋₅-Alkyl ausgewählt ist,
so daß ein polymerisierbares Monomer der Formel I bereitgestellt wird 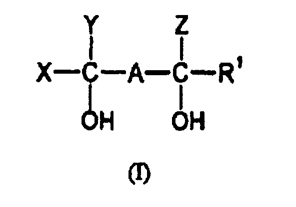 und
(ii) das polymerisierbare Monomer der Formel I polymerisiert unter der Bedingung, daß keine Säure, die in der polymerisierbaren Zusammensetzung vorhanden ist, vor der Polymerisation neutralisiert wird.

## Revendications

1. Procédé pour la préparation d'un monomère polymérisable de formule I 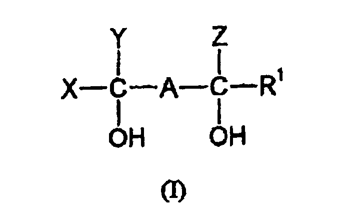 comprenant l'étape de mise en contact d'un composé de formule II 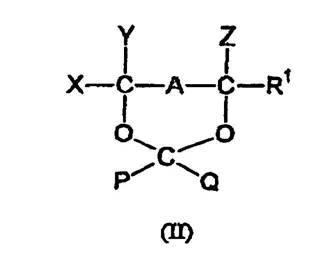 avec un acide immobilisé,
formules dans lesquelles X, Y, Z, P et Q sont choisis indépendamment entre un groupe hydrocarbyle et un atome d'hydrogène, A représente un groupe (CH₂)ₙ dans lequel n est égal à 0 ou 1, et R¹ représente un groupe de formule 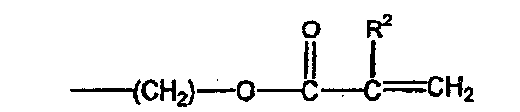 dans laquelle R² est choisi entre H, des groupes méthyle, éthyle, propyle et butyle ; ou bien R¹ représente un groupe de formule III 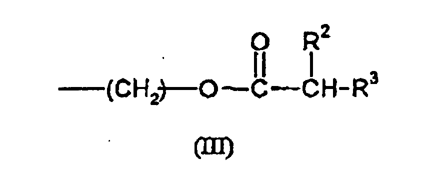 dans laquelle R² est choisi entre des groupes méthyle, éthyle, propyle et butyle, et R³ est choisi parmi des groupes alkyle en C₂ à C₅ insaturés.

2. Procédé suivant la revendication 1, dans lequel l'acide est un acide fort.

3. Procédé suivant la revendication 1 ou 2, dans lequel l'acide est immobilisé sur une résine échangeuse d'ions.

4. Procédé suivant l'une quelconque des revendications précédentes, dans lequel X et Y sont choisis indépendamment entre des groupes hydrocarbonés ayant 1 à 20 atomes de carbone et un atome d'hydrogène.

5. Procédé suivant l'une quelconque des revendications précédentes, dans lequel R¹ représente un groupe de formule 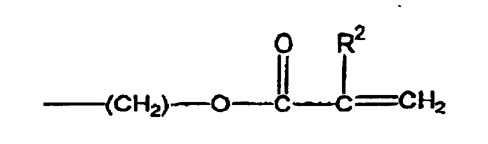 dans laquelle R² représente un groupe CH₃.

6. Procédé suivant l'une quelconque des revendications précédentes, dans lequel X représente H, Y représente H, Z représente H, P représente un groupe CH₃, Q représente un groupe CH₃, N est égal à 0 et R¹ représente un groupe de formule 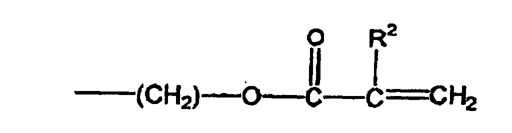 dans laquelle R² représente un groupe CH₃.

7. Procédé suivant l'une quelconque des revendications précédentes, comprenant les étapes consistant à fournir un moyen pour contenir l'acide immobilisé, à mettre en contact l'acide immobilisé avec le composé de formule II et à faire passer un gaz à travers l'acide immobilisé.

8. Procédé suivant la revendication 7, dans lequel le gaz est l'air.

9. Procédé suivant la revendication 7 ou 8, dans lequel l'acide immobilisé est mis en contact avec le composé de formule II en l'absence de solvant organique.

10. Procédé suivant l'une quelconque des revendications 7 à 9, dans lequel le moyen pour contenir l'acide immobilisé comprend un réacteur à lit fluidisé.

11. Procédé suivant l'une quelconque des revendications 7 à 10, le procédé comprenant l'extraction du gaz du moyen pour contenir l'acide immobilisé après le passage du gaz à travers l'acide immobilisé.

12. Procédé suivant l'une quelconque des revendications précédentes, le procédé comprenant en outre l'étape de polymérisation du monomère polymérisable de formule I.

13. Procédé suivant la revendication 12, dans lequel l'acide formé au cours du procédé n'est pas neutralisé avant polymérisation.

14. Procédé suivant la revendication 13, dans lequel l'acide formé est choisi entre l'acide méthacrylique et l'acide acrylique.

15. Procédé suivant la revendication 12, 13 ou 14, comprenant en outre la formation d'un dispositif oculaire à partir du polymère.

16. Procédé pour la préparation d'un polymère, comprenant les étapes consistant
(i) à mettre en contact un composé de formule II 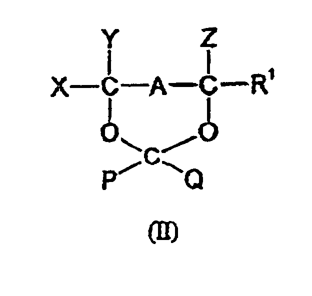 avec un acide immobilisé,
formule dans laquelle X, Y, Z, P et Q sont choisis indépendamment entre un groupe hydrocarbyle et un atome d'hydrogène, A représente un groupe (CH₂)ₙ dans lequel n est égal à 0 ou 1, et R¹ représente un groupe de formule 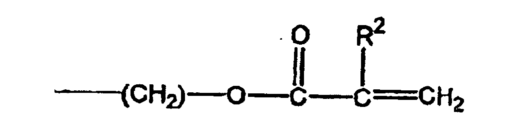 dans laquelle R² est choisi entre H, des groupes méthyle, éthyle, propyle et butyle ; ou bien R¹ représente un groupe de formule III 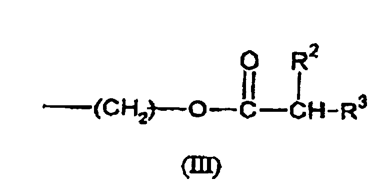 dans laquelle R² est choisi entre des groupes méthyle, éthyle, propyle et butyle, et R³ est choisi parmi des groupes alkyle en C₂ à C₅ insaturés ;
de manière à obtenir un monomère polymérisable de formule I 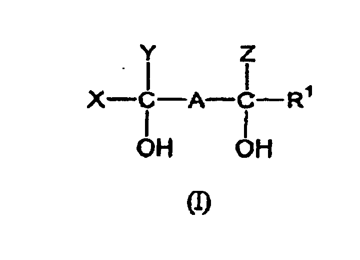 et
(ii) à polymériser le monomère polymérisable de formule I, sous réserve que tout acide présent dans la composition polymérisable ne soit pas neutralisé avant polymérisation.
